# EUROPEAN PATENT APPLICATION

(11) **EP 4 552 550 A1**
(43) Date of publication of application: **14.05.2025**
(21) Application number: 24206507.6
(22) Date of filing: 14.10.2024
(51) Int. Cl.: A61B 3/10, G02B 27/00

(54) **SCANNING LASER OPHTHALMOSCOPE HAVING REDUCED LIGHT REFLECTION**

(30) Priority: 07.11.2023 KR 20230153008
(71) Applicant: Huvitz Co., Ltd., Anyang-si, Gyeonggi-do 14055 (KR)
(72) Inventor: JO, Sunhyung, 14055 Anyang-si (KR); BEAK, Woo Kyung, 14055 Anyang-si (KR)
(74) Representative: Henkel & Partner mbB

(57) **Abstract**

A scanning laser ophthalmoscope capable of reducing reflected light generated from the objective lens is disclosed. The scanning laser ophthalmoscope may include: a light source (10) configured to irradiate measurement light; a scanner (16) configured to sequentially irradiate the measurement light to a desired position of the eye to be examined (5) by adjusting the reflection angle of the measurement light; an objective lens unit (30) configured to focus the measurement light reflected from the scanner (16) to form the focus of the measurement light on the retina of the eye to be examined (5); and a light detector 20 configured to detect retinal reflection light formed by the measurement light being reflected from the retina of the eye to be examined (5), wherein the objective lens unit (30) may include a first objective lens (32) positioned to be inclined at a first angle (a) with the vertical direction of the traveling path of the measurement light, a second objective lens (34) positioned to be inclined at a second angle (b) therewith, and a third objective lens (36) positioned in the vertical direction of the traveling path of the measurement light, and the inclination angle (a) of the first objective lens (32) and the inclination angle (b) of the second objective lens (34) may be formed to be inclined in opposite directions with respect to the vertical direction of the traveling path of the measurement light.

## Description

### CROSS-REFERENCE TO RELATED APPLICATION

This application claims benefit of priority to Korean Patent Application No. 10-2023-0153008 filed on November 07, 2023, the entire contents of which are incorporated herein by reference.

### FIELD OF THE DISCLOSURE

The present disclosure relates to a scanning laser ophthalmoscope (SLO) and, more particularly, to a scanning laser ophthalmoscope capable of reducing reflected light generated from the objective lens.

### BACKGROUND

A scanning laser ophthalmoscope (SLO) is an ophthalmic equipment that projects laser light generated from a light source onto the subject of examination, such as the retina of the eye to be examined, and detects the reflected light reflected from the retina to obtain a retinal image. Retinal imaging tests are useful not only in ophthalmology but also in internal medicine for diagnosing hypertension, diabetes, and neurological diseases.

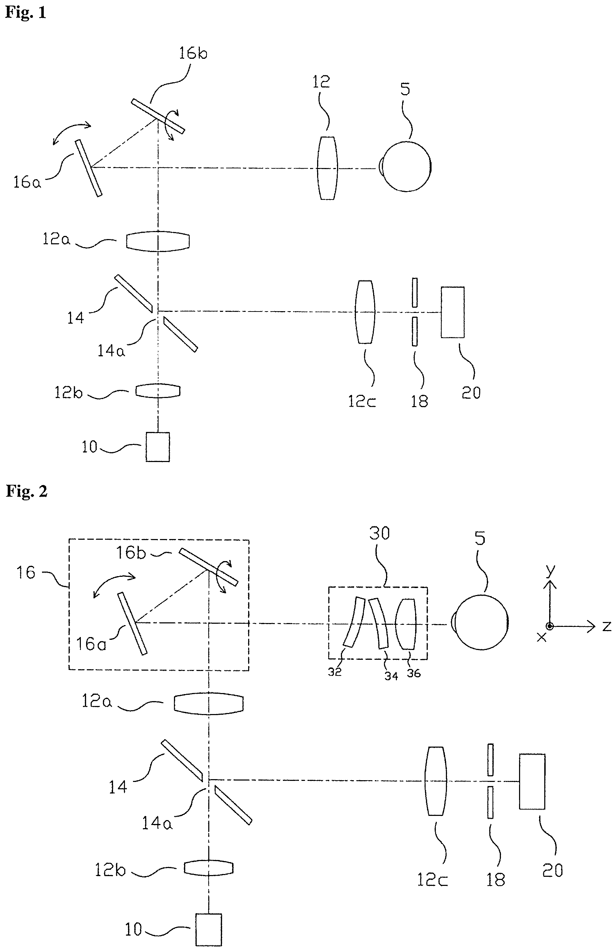

FIG. 1 is a diagram illustrating the configuration of a typical scanning laser ophthalmoscope (SLO). As shown in FIG. 1, in the typical scanning laser ophthalmoscope, laser light (measurement light) is generated from a laser light source 10, and the generated laser light passes through a beam splitter 14, is reflected by reflective mirrors 16a and 16b, and is incident on the retina of the eye to be examined 5. At this time, the reflective mirrors 16a and 16b are rotated by the galvanometer to change the reflection angle of the laser light so as to irradiate the laser light to the desired measurement position of the retina, thereby scanning the observation region of the retina. The reflected light reflected from the retina of the eye to be examined 5, which travels in the opposite direction of the measurement light, is reflected by the reflective mirrors 16a and 16b and the beam splitter 14, passes through the pinhole 18 functioning as a confocal slit, and is then detected by the light detector 20. In the optical system of the scanning laser ophthalmoscope, an objective lens 12 and a plurality of relay lenses 12a, 12b and 12c are used to transmit laser light generated from a laser light source 10 to a point on the retina and transmit laser light reflected from the point on the retina to the light detector 20.

When measuring the retinal surface, the focus of the laser light generated from the laser light source 10 is formed on the retinal surface, and the focus of the reflected light reflected from the retinal surface is formed at the position of the pinhole 18 in front of the light detector 20, so that the focus of the laser light formed on the retina and the focus of the reflected light formed at the position of the pinhole 18 have a conjugate (confocal) relationship. The pinhole 18 serves as a confocal slit that suppresses noise such as unnecessary lens reflection light and scattered light generated by the eye to be examined 5 and the optical system from entering the light detector 20.

In such a confocal scanning laser ophthalmoscope, the retinal surface and the pinhole 18 are to share the focus of the retinal reflection light, so that only the retinal reflection light (retinal image signal) with noise removed is introduced into the light detector 20. However, since multiple lenses 12 and 12a used in the optical system of the scanning laser ophthalmoscope (SLO), especially the central portion of the objective lens 12, are virtually like mirrors on which measurement light is reflected, the measurement light reflected from the objective lens 12 may enter the light detector 20. In this way, to remove noise, that is, lens reflection light reflected from the lenses 12 and 12a of the optical system, a structure such as a hole mirror 14 with a hole 14a, a black dot, or a pinhole is used. However, in the case of using such a noise removal structure, when the diopter of the optical system is corrected according to the refractive power of the eye to be examined 5, a problem may arise in which noise increases further due to a change in the conjugation position of the optical system.

### [Prior Art Documents]

Japanese Patent Publication No. 2016-123467 (Publication date: July 11, 2016)
Japanese Patent Publication No. 2019-118652 (Publication date: July 22, 2019)
European Patent Publication No. 3235421 (Publication date: October 25, 2017)

### SUMMARY OF THE DISCLOSURE

### TECHNICAL OBJECTS

An object of the present disclosure is to provide a scanning laser ophthalmoscope capable of reducing reflected light generated from the objective lens.

Another object of the present disclosure is to provide a scanning laser ophthalmoscope that is capable of improving the signal of the measurement target, i.e., image quality, by suppressing noise generated in the objective lens from entering the light detector.

### TECHNICAL SOLUTION

To achieve the above objects, the present disclosure provides a scanning laser ophthalmoscope, which may include: a light source (10) configured to irradiate measurement light; a scanner (16) configured to sequentially irradiate the measurement light to a desired position of the eye to be examined (5) by adjusting the reflection angle of the measurement light; an objective lens unit (30) configured to focus the measurement light reflected from the scanner (16) to form the focus of the measurement light on the retina of the eye to be examined (5); and a light detector 20 configured to detect retinal reflection light formed by the measurement light being reflected from the retina of the eye to be examined (5), wherein the objective lens unit (30) may include a first objective lens (32) positioned to be inclined at a first angle (a) with the vertical direction of the traveling path of the measurement light, a second objective lens (34) positioned to be inclined at a second angle (b) therewith, and a third objective lens (36) positioned in the vertical direction of the traveling path of the measurement light, and the inclination angle (a) of the first objective lens (32) and the inclination angle (b) of the second objective lens (34) may be formed to be inclined in opposite directions with respect to the vertical direction of the traveling path of the measurement light.

### EFFECTS OF THE DISCLOSURE

The scanning laser ophthalmoscope according to the present disclosure may reduce reflected light generated from the objective lens and suppress noise generated from the objective lens from entering the light detector to thereby improve the signal of the measurement target, i.e., image quality.

### BRIEF DESCRIPTION OF THE DRAWINGS

FIG. 1 is a diagram illustrating the configuration of a typical scanning laser ophthalmoscope.
FIG. 2 is a diagram for explaining the configuration of a scanning laser ophthalmoscope according to an embodiment of the present disclosure.
FIG. 3 is a diagram showing the configuration of an objective lens unit usable in the scanning laser ophthalmoscope according to the present disclosure.
FIG. 4 is a diagram showing the path of measurement light passing through the objective lens unit in the scanning laser ophthalmoscope according to the present disclosure.
FIG. 5 is a diagram depicting a state in which the lens reflection light generated by measurement light being reflected from the objective lens is suppressed from entering the light detector by a first objective lens and a second objective lens in the scanning laser ophthalmoscope according to the present disclosure.

### DETAILED DESCRIPTION OF THE PREFERRED EMBODIMENTS

Hereinafter, various embodiments of the disclosure will be described in detail with reference to the accompanying drawings. Hereinafter, the present disclosure will be described in detail with reference to the accompanying drawings. In the attached drawings, elements that perform the same or similar functions as those in the related art are given the same reference symbols.

FIG. 2 is a diagram for explaining the configuration of a scanning laser ophthalmoscope according to an embodiment of the present disclosure. As shown in FIG. 2, the scanning laser ophthalmoscope according to the present disclosure includes a light source 10 that irradiates measurement light, a scanner 16 that sequentially irradiates the measurement light to a desired position on the eye to be examined 5 by adjusting the reflection angle of the measurement light, an objective lens unit 30 that focuses the measurement light reflected from the scanner 16 to form the focus of the measurement light on the retina of the eye to be examined 5, and a light detector 20 that detects retinal reflection light formed by the measurement light being reflected from the retina of the eye to be examined 5.

The light source 10 is a typical device that preferably generates monochromatic laser measurement light as retinal image measurement light, and generates, for example, red (R), green (G), or blue (B) laser light. The scanner 16 is a typical device that adjusts the reflection angle of the measurement light so that the measurement light is irradiated in sequence to desired positions of the eye to be examined 5. Specifically, the scanner 16 irradiates measurement light through the pupil of the eye to be examined 5 to the retina of the eye to be examined 5 in such a way that the measurement light is sequentially irradiated to each position in the x-y plane of the retina. For example, the scanner 16 may include an x-direction scanner 16b that changes the x-direction irradiation position of the measurement light and a y-direction scanner 16a that changes the y-direction irradiation position of the measurement light, and the scanner 16 may sequentially adjust the positions of the x-direction scanner 16b and the y-direction scanner 16a so that the measurement light is irradiated in sequence to each position of the retina. That is, the scanner 16 enables the measurement light to sequentially scan the retinal surface of the eye to be examined 5.

The measurement light reflected from the scanner 16 is focused by the objective lens unit 30 to form the focus of the measurement light on the retina of the eye to be examined 5. The light detector 20 detects the reflected light formed by the measurement light being reflected from the retina of the eye to be examined 5. When the measurement light is red (R) laser light, the reflected light reflected from the retina of the eye to be examined 5 includes red color information of the reflection position. Hence, when the retinal surface of the eye to be examined 5 is scanned with red (R) laser light, a red image of the retinal surface may be obtained. Likewise, when the retinal surface is scanned with green (G) laser light, a green image of the retinal surface may be obtained; when the retinal surface is scanned with blue (B) laser light, a blue image of the retinal surface may be obtained; and when these are combined, a color image of the retinal surface may be obtained. The reflected light reflected from the retina of the eye to be examined 5 passes through the objective lens unit 30, is reflected from the scanner 16 and beam splitter 14, and is guided to the light detector 20.

As shown in FIG. 2, a hole 14a may be formed in the beam splitter 14 to transmit the measurement light. In addition, the scanning laser ophthalmoscope according to the present disclosure may further include, as typical optical system elements, a collimation lens 12b for converting laser light into parallel light, a diopter lens 12a for focusing and transmitting laser light, a focusing lens 12c for focusing reflected light, and a pinhole 18 for removing noise components from reflected light.

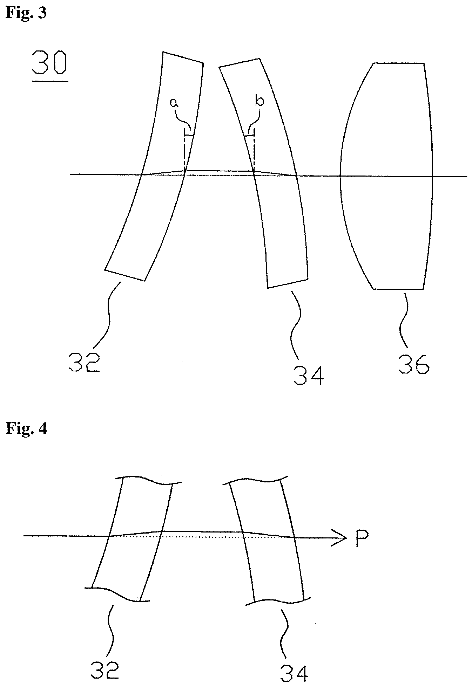

FIG. 3 is a diagram showing the configuration of the objective lens unit 30 usable in the scanning laser ophthalmoscope according to the present disclosure, and FIG. 4 is a diagram illustrating the path of the measurement light passing through the objective lens unit 30 in the scanning laser ophthalmoscope according to the present disclosure. As shown in FIG. 3, the objective lens unit 30 usable in the scanning laser ophthalmoscope according to the present disclosure includes a first objective lens 32 positioned to be inclined at a first angle (a) with the vertical direction of the traveling path of the measurement light, a second objective lens 34 positioned to be inclined at a second angle (b) therewith, and a third objective lens 36 positioned in the vertical direction of the traveling path of the measurement light. Here, the inclination angle (a) of the first objective lens 32 and the inclination angle (b) of the second objective lens 34 are formed to be inclined in opposite directions with respect to the vertical direction of the traveling path of the measurement light. That is, if the first objective lens 32 is inclined clockwise with respect to the vertical direction of the traveling path of the measurement light, the second objective lens 34 is inclined counterclockwise with respect to the vertical direction of the traveling path of the measurement light. Conversely, if the first objective lens 32 is inclined counterclockwise with respect to the vertical direction of the traveling path of the measurement light, the second objective lens 34 is inclined clockwise with respect to the vertical direction of the traveling path of the measurement light.

As shown in FIG. 4, when the central lens surface of the first objective lens 32 is inclined clockwise with respect to the vertical direction of the traveling path of the measurement light, the measurement light is refracted in the first objective lens 32, and the actual trajectory of the measurement light (P, indicated by a solid line in FIG. 4) is refracted upward with respect to the optical axis of the measurement light (indicated by a dotted line in FIG. 4) and then travels straight. The measurement light, which is refracted upward and then travels straight, is refracted downward in the second objective lens 34, whose central lens surface is inclined counterclockwise with respect to the vertical direction of the traveling path of the measurement light, and then travels straight, so that the traveling path of the measurement light returns to its original trajectory. Meanwhile, as the central lens surface of the third objective lens 36 is positioned in the vertical direction of the traveling path of the measurement light, the trajectory of the measurement light does not change. In this way, the first objective lens 32 and the second objective lens 34 should rotate in opposite directions with respect to the vertical direction of the traveling path of the measurement light, so that the traveling path of the measurement light is maintained and the optical components used in the scanning laser ophthalmoscope may be easily aligned.

Therefore, in the present disclosure, to maintain the same traveling path of the measurement light before and after passing through the objective lens unit 30, it is preferable that the inclination angle (a) of the first objective lens 32 and the inclination angle (b) of the second objective lens 34 are formed at the same angle in opposite directions with respect to the vertical direction of the traveling path of the measurement light. In this case, it is preferable that the first objective lens 32 and the second objective lens 34 have the same thickness, shape, and material. In the case where the first objective lens 32 and the second objective lens 34 are different in at least one of thickness, shape, and material, if the refractive indices of the first objective lens 32 and the second objective lens 34 are adjusted to be the same, the traveling path of the measurement light before and after passing through the objective lens unit 30 may be maintained to be the same. In addition, when the refractive indices of the first objective lens 32 and the second objective lens 34 are different, the inclination angle (a) of the first objective lens 32 and the inclination angle (b) of the second objective lens 34 may be adjusted differently to maintain the same traveling path of the measurement light before and after passing through the objective lens unit 30.

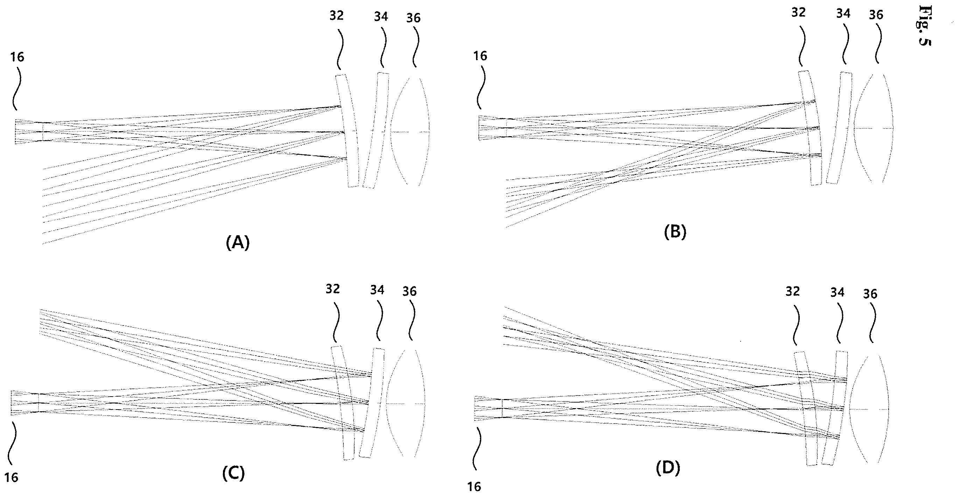

FIG. 5 is a diagram depicting a state in which noise, i.e., lens reflection light generated by the measurement light being reflected from the objective lens, is suppressed from entering the light detector 20 by the first objective lens 32 and the second objective lens 34 which are positioned inclined so as to face each other in the scanning laser ophthalmoscope according to the present disclosure. As shown in part (A) of FIG. 5, the measurement light reflected from the scanner 16 is reflected from the front surface (first surface) of the first objective lens 32 being inclined counterclockwise with respect to the vertical direction of the traveling path of the measurement light, and the reflected light formed at that time proceeds under the traveling path of the measurement light. As shown in part (B) of FIG. 5, the measurement light passing through the first objective lens 32 is reflected again from the rear surface (second surface) of the first objective lens 32 being inclined counterclockwise with respect to the vertical direction of the traveling path of the measurement light, and the reflected light formed at that time also proceeds under the traveling path of the measurement light. As shown in part (C) of FIG. 5, the measurement light having passed through the first objective lens 32 is reflected again from the front surface (first surface) of the second objective lens 34 being inclined clockwise with respect to the vertical direction of the traveling path of the measurement light, and the reflected light formed at that time proceeds above the traveling path of the measurement light. As shown in part (D) of FIG. 5, the measurement light passing through the second objective lens 34 is reflected again from the rear surface (second surface) of the second objective lens 34 being inclined clockwise with respect to the vertical direction of the traveling path of the measurement light, and the reflected light formed at that time also proceeds above the traveling path of the measurement light. Hence, the lens reflection light reflected from the first objective lens 32 and the second objective lens 34 is reflected with being dispersed upward and downward with respect to the traveling path of the measurement light, which may minimize the influence of the reflection light on the optical system of the scanning laser ophthalmoscope and suppress the reflection light from entering the light detector 20.

Referring back to FIGS. 2, 3 and 5, the inclination angle (a) of the first objective lens 32 and the inclination angle (b) of the second objective lens 34 may be set so that the lens reflection light reflected from each surface of the first objective lens 32 and the second objective lens 34 does not enter the scanner 16. The traveling path of the lens reflection light varies depending on the curvature (R) of the front and rear surfaces of the first objective lens 32, for example, front surface curvature (R) of -200 to -500 mm, rear surface curvature (R) of -100 to 200 mm, and the curvature (R) of the front and rear surface of the second objective lens 34, for example, front surface curvature (R) of -200 mm to infinity, rear surface curvature (R) of -100 to 200 mm, or the like. Therefore, considering the curvature of the first objective lens 32 and the second objective lens 34, the distance between the first and second objective lenses 32 and 34 and the scanner 16, or the like, the inclination angle (a) of the first objective lens 32 and the inclination angle (b) of the second objective lens 34 may be appropriately set so that the lens reflection light reflected from each surface of the first objective lens 32 and the second objective lens 34 does not enter the scanner 16.

Although the present disclosure has been described with reference to the attached drawings and exemplary embodiments, the present disclosure is not limited to the contents illustrated in the drawings and the embodiments described above but should be interpreted comprehensively to include all modifications of the exemplary embodiments, and equivalent structures and functions. Although reference symbols are used in the claims below to aid understanding, the scope of the claims is not limited by those reference symbols and the contents depicted in the drawings.

## Claims

1. A scanning laser ophthalmoscope comprising:
a light source (10) configured to irradiate measurement light;
a scanner (16) configured to sequentially irradiate the measurement light to a desired position of an eye to be examined (5) by adjusting a reflection angle of the measurement light;
an objective lens unit (30) configured to focus the measurement light reflected from the scanner (16) to form a focus of the measurement light on a retina of the eye to be examined (5); and
a light detector (20) configured to detect retinal reflection light formed by the measurement light being reflected from the retina of the eye to be examined (5),
wherein the objective lens unit (30) includes a first objective lens (32) positioned to be inclined at a first angle (a) with a vertical direction of a traveling path of the measurement light, a second objective lens (34) positioned to be inclined at a second angle (b) therewith, and a third objective lens (36) positioned in the vertical direction of the traveling path of the measurement light, and the inclination angle (a) of the first objective lens (32) and the inclination angle (b) of the second objective lens (34) are formed to be inclined in opposite directions with respect to the vertical direction of the traveling path of the measurement light.

2. The scanning laser ophthalmoscope of claim 1, wherein the inclination angle (a) of the first objective lens (32) and the inclination angle (b) of the second objective lens (34) are formed at the same angle in opposite directions with respect to the vertical direction of the traveling path of the measurement light so as to maintain the same traveling path of the measurement light before and after passing through the objective lens unit (30).

3. The scanning laser ophthalmoscope of claim 2, wherein the first objective lens (32) and the second objective lens (34) have the same thickness, shape, and material.

4. The scanning laser ophthalmoscope of claim 1, wherein the first objective lens (32) and the second objective lens (34) are different in at least one of thickness, shape and material, and the refractive indexes of the first objective lens (32) and the second objective lens (34) are adjusted to be the same so as to maintain the same traveling path of the measurement light before and after passing through the objective lens unit (30).

5. The scanning laser ophthalmoscope of claim 1, wherein the refractive indexes of the first objective lens (32) and the second objective lens (34) are different, and the inclination angle (a) of the first objective lens (32) and the inclination angle (b) of the second objective lens (34) are adjusted differently so as to maintain the same traveling path of the measurement light before and after passing through the objective lens unit (30).
